# FASCICULE DE BREVET EUROPEEN

(11) **EP 1 351 907 B1**
(45) Date de publication et mention de la délivrance du brevet: **15.11.2006**
(21) Numéro de dépôt: 01270513.3
(22) Date de dépôt: 10.12.2001
(51) Int. Cl.: C07C 15/107, C07D 307/80

(54) **CHLORHYDRATE DU 2-BUTYL-3-(4-'3-(DIBUTYLAMINO)PROPOXY]BENZOYL)-5-NITRO-BENZOFURANE ET SA PREPARATION**
2-BUTYL-3-(4-3(DIBUTYLAMINO)PROPOXY] BENZOYL)-5-NITROBENZOFURAN-HYDROCHLORID UND DESSEN HERSTELLUNG
2-BUTYL-3-(4- 3(DIBUTYLAMINO)PROPOXY BENZOYL)-5-NITRO-BENZOFURAN HYDROCHLORIDE AND PREPARATION THEREOF

(30) Priorité: 11.12.2000 FR 0016069
(43) Date de publication de la demande: 15.10.2003
(73) Titulaire: Sanofi-Aventis, 75013 Paris (FR)
(72) Inventeur: BIARD, Michel, F-04200 Sisteron (FR)
(74) Mandataire: Weber, Mathieu
(86) Numéro de dépôt international: PCT/FR2001/003900
(87) Numéro de publication internationale: WO 2002/048078

(56) Documents cités:
- EP-A- 0 471 609
- Y. OSHISHI ET AL.: "Antibacterial activity and Polarographic Half-Wave Reduction Potential of 2-Nitrobenzo[b]furans" CHEM. PHARM. BULL, vol. 33, no. 7, 1985, pages 2854-2861, XP001056510

## Description

La présente invention se rapporte, d'une manière générale, à un dérivé aminoalkoxybenzoyle sous forme de sel, à son procédé de préparation ainsi qu'à son utilisation comme intermédiaire de synthèse.

Plus précisément, l'invention a pour objet un procédé de préparation du chlorhydrate du 2-butyl-3-(4-[3-(dibutylamino)propoxy]benzoyl)-5-nitro-benzofurane de formule : 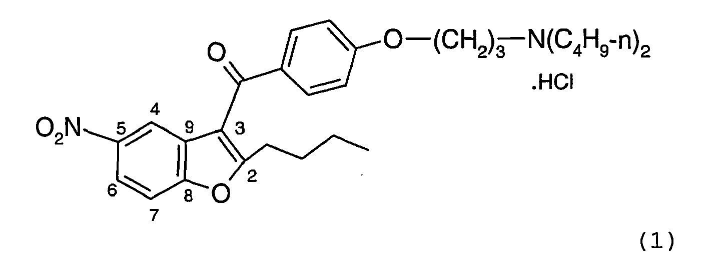 ci-après dénommé « chlorhydrate du Composé A ». Ce composé s'est révélé particulièrement utile comme produit intermédiaire pour la préparation du 2-butyl-3-(4-[3-(dibutylamino)propoxy]benzoyl)-5-nitro-benzofurane ci-après dénommé « Composé A ».

Ce dernier peut être lui-même largement utilisé comme intermédiaire dans la préparation de différents produits notamment pour la synthèse finale de dérivés d'aminoalkoxybenzoyl-benzofurane en particulier du 2-butyl-3-(4-[3-(dibutylamino)propoxy]benzoyl)-5-méthanesulfonamido-benzofurane communément appelé dronédarone ainsi que de ses sels pharmaceutiquement acceptables.

Ce dérivé de méthanesulfonamido-benzofurane ainsi que ses sels pharmaceutiquement acceptables ont été décrits dans le brevet EP0471609 de même que ses applications thérapeutiques. Dans le domaine cardiovasculaire, ce composé s'est montré particulièrement intéressant notamment comme agent antiarythmique.

On a également rapporté dans le brevet EP0471609, cité précédemment, un procédé pour la préparation de dérivés 3-[4-(aminoalkoxy)benzoyl]benzofurane ou benzo [b] thiophène par fixation d'une chaîne aminoalkoxybenzoyle sur un dérivé de benzofurane ou de benzo [b] thiophène, procédé selon lequel on ajoute d'abord, sur le dérivé de benzofurane ou de benzo [b] thiophène en question, un groupement benzoyle comportant en position para un oxygène protégé par un groupement méthyle, on déprotège pour regénérer la fonction hydroxyle et on introduit finalement la chaîne aminoalkyle souhaitée.

Plus spécifiquement, ce procédé appliqué à la préparation du Composé A comporte la suite d'étapes ci-après :
a) réaction du 2-butyl-5-nitro-benzofurane avec le chlorure d'anisoyle en présence de tétrachlorure d'étain selon les conditions de la réaction de Friedel-Crafts et hydrolyse pour former le 2-butyl-3-(4-méthoxy-benzoyl)-5-nitro-benzofurane,
b) déméthylation du composé ainsi obtenu en présence de 2,25 équivalents molaires de chlorure d'aluminium et hydrolyse pour former le 2-butyl-3-(4-hydroxy-benzoyl)-5-nitro-benzofurane,
c) condensation du composé ainsi obtenu avec le 1-chloro-3-dibutylamino-propane en présence de carbonate de potassium, pour donner le Composé A souhaité.

Toutefois, ce procédé n'est pas sans présenter certains inconvénients en raison notamment de l'utilisation du chlorure d'aluminium. En effet, la mise en oeuvre de ce procédé à l'échelle industrielle provoque d'importants rejets d'hydroxyde d'aluminium dont le traitement, en vue d'éviter des problèmes de pollution, s'avère onéreux. En outre, l'usage du 2-butyl-3-(4-methoxy-benzoyl)-5-nitro-benzofurane devrait être évité en raison de ses propriétés mutagènes.

Enfin, le composé désiré n'est produit, selon ce procédé en trois étapes, qu'avec un rendement maximum de 60% à partir du 2-butyl-5-nitro-benzofurane.

Des étapes analogues aux étapes a) et b) ci-dessus sont décrites dans l'article de Y. OSHISHI et al, « Antibacterial Activity and Polarographic Half-Wave Réduction Potential of 2-Nitro-benzo[b]furans », CHEM. PHARM. BULL., vol.33, no.7, 1985, pages 2854-2861. Ce document décrit en particulier un procédé pour la préparation du composé 2-ethyl-3-4'-hydroxybenzoyl-5-nitrobenzo[b]furane (« composé (7d) ») à partir du 2-ethyl-5-nitro-benzofurane (« composé (7c) »), en formant le composé intermédiaire 2-ethyl-3-(4-méthoxy-benzoyl)-5-nitro-benzofurane.

La recherche d'un procédé industriel pour la préparation du Composé A mettant en oeuvre un nombre minimum d'étapes de synthèse à partir du 2-butyl-5-nitro-benzofurane tout en évitant l'utilisation du chlorure d'aluminium reste, par conséquent, d'un intérêt incontestable.

On a rapporté dans J.Med.Chem. 1984,27,1057-1066 une méthode plus convergente pour fixer une chaîne aminoalkoxybenzoyle sur un dérivé de benzo [b] thiophène sans transiter par une étape de protection de la fonction hydroxyle. Toutefois, ce procédé propose encore, à la page 1064, l'utilisation du chlorure d'aluminium en quantités particulièrement importantes puisque de l'ordre de 9 équivalents molaires. Selon cette méthode, on condense dans une phase organique constituée de dichloréthane le dérivé de benzo [b] thiophène en question avec le chlorhydrate du chlorure du dérivé aminoalkoxybenzoyle et ce, en présence de chlorure d'aluminium. Après hydrolyse, le chlorhydrate du dérivé 3-[4-(aminoalkoxy)benzoyl]benzo [b] thiophène désiré est récupéré en partie de la phase organique et en partie de la phase aqueuse par trois extractions au chloroforme puis traité par l'hydroxyde de sodium.

Dans le cadre de l'élaboration de la présente invention, on a appliqué ce procédé à la préparation du Composé A par mise en oeuvre des étapes suivantes :
- traitement du 2-butyl-5-nitro-benzofurane au moyen du chlorhydrate du chlorure de 4-[3-(dibutylamino)propoxy]benzoyle en présence de 9 équivalents molaires de chlorure d'aluminium et ce, dans une phase organique,
- hydrolyse, récupération du chlorhydrate du Composé A et traitement par l'hydroxyde de sodium pour former le Composé A souhaité.

Cependant, ce procédé s'est avéré inadapté au niveau industriel en raison d'une part de l'énorme quantité d'hydroxyde d'aluminium ainsi produit et d'autre part du taux important d'impuretés récoltées et, par conséquent, du faible rendement fourni en Composé A souhaité (20 à 30%).

Or, de façon surprenante, on a trouvé qu'il est possible à partir du 2-butyl-5-nitro-benzofurane et en utilisant des quantités appropriées d'un acide de Lewis dans une réaction de Friedel-Crafts d'obtenir le chlorhydrate du Composé A avec un rendement important puisque supérieur à 90 %, ce chlorhydrate pouvant être récupéré de manière remarquablement avantageuse puisqu'on le retrouve en quasi totalité, non pas dans la phase aqueuse comme on aurait pu le prévoir, mais dans la phase organique utilisée, ce qui évite la nécessité de procéder à plusieurs extractions de cette même phase aqueuse comme dans le procédé antérieur.

En outre, ce chlorhydrate peut être mis en oeuvre avec grande facilité pour la préparation du Composé A qui peut être produit, selon l'invention, avec des rendements supérieurs à 95 %.

Selon l'invention, on prépare le chlorhydrate du Composé A en faisant réagir, dans une phase organique, le 2-butyl-5-nitro-benzofurane avec le chlorhydrate du chlorure de 4-[3-(dibutylamino)propoxy]benzoyle et ce, en présence d'un acide de Lewis comme catalyseur et, en hydrolysant pour former le composé désiré que l'on récupère dans la phase organique.

La réaction, entreprise selon les conditions de la réaction de Friedel-Crafts, est effectuée habituellement à la température ambiante et dans une phase organique constituée d'un ou de plusieurs solvants choisis parmi des hydrocarbures halogénés ou non de préférence de type aliphatique, alicyclique ou aromatique. Généralement, on utilise des hydrocarbures halogénés, de préférence chlorés, de type aliphatique, alicyclique ou aromatique, tels que par exemple le dichlorométhane, le dichloréthane ou le chlorobenzène.

En outre, l'acide de Lewis peut être le chlorure d'aluminium, le chlorure de zinc, le trifluorure de bore, le chlorure stannique, le tétrachlorure de titane ou, de préférence, le chlorure ferrique. On peut utiliser un mélange de ceux-ci. Cet acide de Lewis est utilisé selon des quantités n'excédant pas 3 équivalents molaires, en particulier à raison de 2 à 3 équivalents molaires de préférence 2,5 équivalents molaires.

Enfin, le chlorhydrate du chlorure de 4-[3-(dibutylamino)propoxy]benzoyle est mis en oeuvre en léger excès quantitatif tel que par exemple selon des quantités de l'ordre de 1 à 1,3 équivalent molaire.

Ce chlorhydrate de chlorure est un produit nouveau au même titre que d'autres composés intervenant dans la préparation du chlorhydrate du Composé A.

Toutefois, le brevet EP0471609 cite ponctuellement dans son exemple 28 le composé chlorhydrate de chloro-1 (di-n-butylamino-3 propoxy)-4 benzoyle pour son utilisation dans la synthèse de l'oxalate acide de n-butyl-2[(di-n-butylamino-3 propoxy)-4 benzoyl]-3 carbéthoxy-7 indolizine.

En conséquence, l'invention aussi décrit des dérivés benzoyles de formule générale : 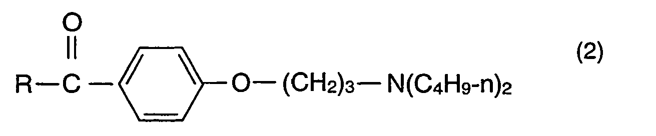 ainsi qu'à leur chlorhydrate, dans laquelle R représente le chlore ou un groupement -OR₁ dans lequel R₁ représente l'hydrogène ou un groupement alkyle en C₁-C₄, à l'exclusion du chlorhydrate de chloro-1 (di-n-butylamino-3 propoxy)-4 benzoyle, en tant que produits industriels nouveaux utiles notamment comme intermédiaires de synthèse, par exemple pour la préparation du chlorhydrate du Composé A.

A titre d'exemple, on peut citer :
- le chlorhydrate de l'acide 4-[3-(dibutylamino)propoxy]benzoïque,
- le chlorhydrate du chlorure de 4-[3-(dibutylamino)propoxy]benzoyle,
- le 4-[3-(dibutylamino)propoxy]benzoate de méthyle.

Le chlorhydrate du chlorure de 4-[3-(dibutylamino)propoxy]benzoyle ainsi que les autres composés de formule (2) peuvent être préparés selon la suite d'étapes ci-après :
a) on fait réagir le 1-dibutylamino-3-chloro-propane avec un p-hydroxybenzoate d'alkyle en C₁-C₄ par exemple le p-hydroxybenzoate de méthyle et ce, en présence d'un agent basique tel qu'un carbonate de métal alcalin par exemple le carbonate de potassium, pour obtenir un 4-[3-(dibutylamino)propoxy]benzoate d'alkyle en C₁-C₄ de formule (2) par exemple le 4-[3-(dibutylamino)propoxy]benzoate de méthyle,
b) on saponifie l'ester ainsi obtenu, en présence d'un hydroxyde de métal alcalin par exemple l'hydroxyde de sodium puis on traite le sel ainsi formé au moyen d'acide chlorhydrique pour donner le chlorhydrate de l'acide 4-[3-(dibutylamino)propoxy]benzoïque,
c) on traite alors le chlorhydrate ainsi formé au moyen d'un agent de chloration par exemple le chlorure de thionyle, pour obtenir le composé désiré.

Comme mentionné précédemment, le chlorhydrate du Composé A peut donner accès au Composé A.

Composé A est préparé par traitement de son chlorhydrate au moyen d'un agent basique tel qu'un hydroxyde de métal alcalin, par exemple l'hydroxyde de sodium, un carbonate de métal alcalin ou un hydrogénocarbonate de métal alcalin, pour obtenir le composé désiré. Préférentiellement, on utilise un hydrogénocarbonate de métal alcalin tel que l'hydrogénocarbonate de sodium. Selon une mise en oeuvre préférée, on prépare le Composé A sans isolement de son chlorhydrate formé transitoirement, c'est-à-dire dans le milieu même où ce chlorhydrate est préparé.

En conséquence, on prépare le Composé A moyennant un procédé selon lequel dans une phase organique, constituée d'un ou de plusieurs solvants choisis parmi des hydrocarbures halogénés ou non, on fait réagir le 2-butyl-5-nitro-benzofurane avec le chlorhydrate du chlorure de 4-[3-(dibutylamino)propoxy]benzoyle et ce, en présence d'un maximum de 3 équivalents molaires d'un acide de Lewis, comme catalyseur, et on hydrolyse pour obtenir transitoirement et sans isolement le chlorhydrate du Composé A que l'on récupère dans la phase organique, et que l'on traite au moyen d'un agent basique, ce qui fournit le Composé A désiré.

Cette méthode est particulièrement avantageuse puisqu'elle permet d'obtenir le Composé A directement à partir du 2-butyl-5-nitro-benzofurane sans aucun isolement d'intermédiaire de synthèse et selon des rendements importants puisque de l'ordre de 97 % à partir du dérivé nitro de départ.

Comme indiqué précédemment, le chlorhydrate du Composé A peut être utilisé pour la préparation de la dronédarone et de ses sels pharmaceutiquement acceptables.

Le chlorhydrate du Composé A est un intermédiaire pour la synthèse finale de la dronédarone et de ses sels pharmaceutiquement acceptables.

Par exemple, on peut préparer ce composé et ses sels au départ du Composé A lui-même obtenu selon l'invention à partir de son chlorhydrate, par mise en oeuvre d'un procédé comportant la suite d'étapes ci-après :
a) dans une phase organique, constituée d'un ou de plusieurs solvants choisis parmi des hydrocarbures halogénés ou non, on fait réagir le 2-butyl-5-nitro-benzofurane avec le chlorhydrate du chlorure de 4-[3-(dibutylamino)propoxy]benzoyle et ce, en présence d'un maximum de 3 équivalents molaires d'un acide de Lewis, comme catalyseur, on hydrolyse pour obtenir transitoirement et sans isolement le chlorhydrate du Composé A que l'on récupère dans la phase organique et que l'on traite au moyen d'un agent basique, pour former le Composé A,
b) on hydrogène le Composé A obtenu en présence d'un catalyseur approprié tel que l'oxyde de platine, ce qui fournit le 5-amino-2-butyl-3-(4-[3-(dibutylamino)propoxy]benzoyl-benzofurane,
c) on fait réagir le dérivé de 5-aminobenzofurane ainsi obtenu avec le chlorure de méthanesulfonyle ou l'anhydride méthanesulfonique, la réaction ayant lieu en présence d'un accepteur d'acide tel que la triéthylamine, ce qui fournit la dronédarone que l'on fait réagir, si on le désire, avec un acide organique ou inorganique pour former un sel pharmaceutiquement acceptable de ce composé.
Les étapes (c) et (d) ci-dessus sont des étapes connues ayant été décrites dans le brevet EP0471609 cité précédemment.

Les exemples non limitatifs suivants illustrent l'invention.

Dans ces exemples, les abréviations utilisées possèdent les significations rapportées ci-dessous :
R.M.N. : résonance magnétique nucléaire
C.L.H.P. : chromatographie liquide haute pression

### PREPARATION

### A. 1-Dibutylamino-3-chloro-propane

Dans un réacteur de 1 l, on introduit 288,4 g (3,392 moles) d'ammoniaque à 20% puis on ajoute, en 10 minutes et à température ambiante (22 ±2°C), 618 g (1,696 mole) de chlorhydrate de 1-dibutylamino-3-chloro-propane (titre 66,5 %). On agite durant 45 minutes à température ambiante et on laisse décanter pendant 30 minutes. On élimine la phase aqueuse inférieure (pH=11) et, avec 300 ml d'eau désionisée, on effectue à température ambiante un lavage de la phase organique. On agite durant 30 minutes, on décante pendant 30 minutes et on soutire la phase aqueuse inférieure (pH=9).

De cette manière, on recueille 346,3 g de composé souhaité. Rendement : 99,4 %

### EXEMPLE 1

### 4-[3-(Dibutylamino)propoxy]benzoate de méthyle

Dans un ballon de 2 l, on introduit 200 g (1,3 mole) de p-hydroxybenzoate de méthyle et 1,6 l de N,N-diméthylformamide. On agite le mélange et on y ajoute 232g (1,66 mole) de carbonate de potassium. On chauffe à 100°C puis on introduit, en 10 minutes, le 1-dibutylamino-3-chloro-propane préparé à l'étape **A.** précédente. On maintient le milieu réactionnel durant 1 heure à 100 ± 2°C puis on refroidit à 25°C. On filtre les sels minéraux, on rince avec 2 fois 50 ml de N,N-diméthylformamide et on concentre le filtrat à l'évaporateur rotatif jusqu'à la température de 85°C et la pression de 5 mmHg (666,61 Pa).

De cette manière, on obtient 472,7 g de produit désiré sous forme d'une huile limpide jaune.
Pureté (CLHP)
   Composé désiré : 99,7 %
   p-Hydroxybenzoate de méthyle : 0,1 %
Spectre R.M.N. (300 MHz)
Solvant : CDCl₃
Concentration : 40 mg/ml
Température d'analyse : 300K

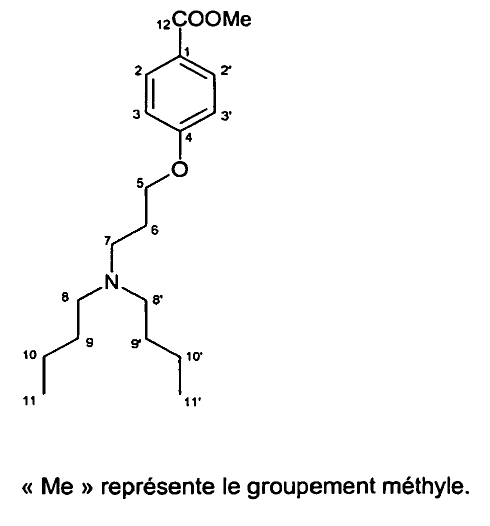

| **Placements chimiques δ ± 0,01 ppm** | **Multiplicité** | **Intégration** | **Constantes de couplage \|J\| ± 0,5 Hz** | **Attribution** |
|---|---|---|---|---|
| 7,97 | Doublet | 2 | ³J_{H-H}≈ 9,0 | H(2) et H(2') |
| 6,90 | Doublet | 2 | ³J_{HH} ≈ 9,0 | H(3) et H(3') |
| 4,06 | Triplet | 2 | ³J_{H-H}≈ 6,5 | OCH₂(5) |
| 3,87 | Singulet | 3 | - | OCH₃ |
| 2,57 | Triplet | 2 | ³J_{H-H} ≈ 7,0 | CH₂(7) |
| 2,40 | Triplet | 4 | ³J_{H-H} ≈ 7,0 | CH₂(8) et CH₂(8') |
| 1,90 | Quintuplet | 2 | ³J_{H-H} ≈ 7,0 | CH₂(6) |
| 1,39 | Multiplet (Quintuplet déformé ) | 4 | - | CH₂(9) et CH₂(9') |
| 1,28 | Multiplet (Sextuplet déformé) | 4 | ³J_{H-H} ≈ 7,0 | CH₂(10) et CH₂(10') |
| 0,87 | Triplet | 6 | ³J_{H-H}≈ 7,0 | CH₃(11) et CH₃(11') |

### EXEMPLE 2

### Chlorhydrate de l'acide 4-[3-(dibutylamino)propoxy]benzoïque

Dans un ballon de 2 l, on introduit 436,3 g de 4-[3-(dibutylamino)propoxy]benzoate de méthyle et 1,092 l de méthanol. On agite le mélange et on introduit, en environ 5 minutes, 360 g (1,8 mole) d'hydroxyde de sodium à 20 %.
On chauffe à 65°C durant environ 30 minutes et on maintient à cette température pendant 2 heures. On refroidit le milieu réactionnel à 30°C et on concentre à l'évaporateur rotatif (température du bain : 30°C, pression 30 mmHg (3999,67 Pa)), ce qui fournit 937 g de résidu que l'on dilue par ajout de 2,8 1 d'eau désionisée. On refroidit la solution à 10 ± 2°C puis, sans dépasser 20°C, on introduit 260 ml (environ 3 moles) d'acide chlorhydrique à 36%.
On vérifie que le pH est inférieur à 1 puis on refroidit la suspension à 10 ± 2°C. On maintient cette température pendant 30 minutes, on essore les cristaux formés et on lave le gâteau avec 2 fois 200 ml d'eau désionisée. On sèche ensuite en étuve ventilée à 50°C jusqu'à poids constant (24 heures).

De cette manière, on obtient 416,2 g de composé désiré sous forme d'une poudre.
Rendement : 100 %.
Pureté (CLHP)
   Composé désiré : 99,5 %
   4-[3-(Dibutylamino)propoxy]benzoate de méthyle : 0,1 %
Spectre R.M.N. (300 MHz)
Solvant : CDCl₃
Concentration : 40 mg/ml
Température d'analyse : 300 K

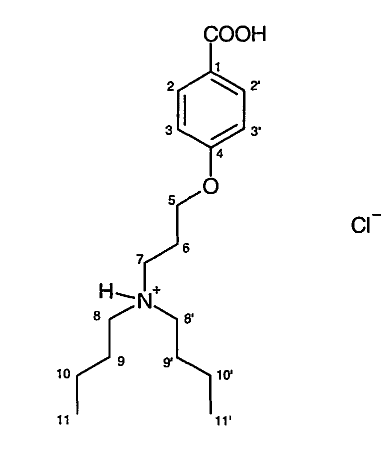

| **Placements chimiques δ ± 0,01 ppm** | **Multiplicité** | **Intégration** | **Constantes de couplage \|J\| ± 0,5 Hz** | **Attribution** |
|---|---|---|---|---|
| 11, 76 et 10,31 | 2 Singulets larges | 2 | - | NH⁺ et COOH |
| 7,93 | Doublet | 2 | ³J_{H-H} ≈ 8,5 | H(2) et H(2') |
| 6,81 | Doublet | 2 | ³J_{H-H} ≈ 8,5 | H(3) et H(3') |
| 4,10 | Triplet | 2 | ³J_{H-H}≈ 5,5 | OCH₂(5) |
| 3,26 | Multiplet | 2 | - | CH₂(7) |
| 3,04 | Multiplet | 4 | - | CH₂(8) et CH₂(8') |
| 2,38 | Multiplet | 2 | - | CH₂(6) |
| 1,78 | Multiplet | 4 | - | CH₂(9) et CH₂(9') |
| 1,37 | Sextuplet | 4 | - | CH₂(10) et CH₂(10') |
| 0,93 | Triplet | 6 | - | CH₃(11) et CH₃(11') |

### EXEMPLE 3

### Chlorhydrate du chlorure de 4-[3-(dibutylamino)

### propoxy]benzoyle

Dans un ballon, on introduit 63,3 g (0,184 mole) de chlorhydrate de l'acide 4-[3-(dibutylamino)propoxy]benzoïque, 300 ml de chlorobenzène et 2 gouttes de N,N-diméthylformamide. Tout en maintenant le mélange sous atmosphère inerte, on introduit, en environ 45 minutes, 43,8 g (0,368 mole) de chlorure de thionyle. On maintient durant 1 heure à 85 ± 1 °C puis on distille sous vide progressif environ 115 g d'un mélange de chlorobenzène et de chlorure de thionyle.

De cette manière, on obtient le composé désiré sous forme d'une poudre et de blocs jaune pâle.
Spectre R.M.N.
Solvant : CDCl₃
Concentration : 40 mg/ml
Température d'analyse : 300K

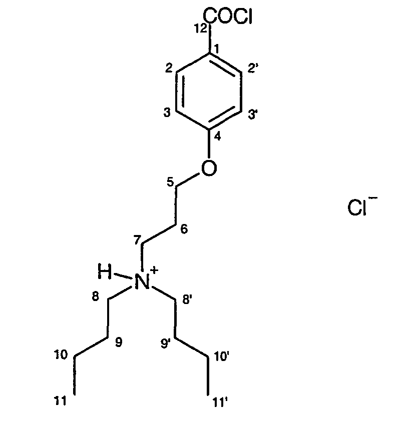

| **Placements chimiques δ ± 0,01 ppm** | **Multiplicité** | **Intégration** | **Constantes de couplage \|J\| ± 0,5 Hz** | **Attribution** |
|---|---|---|---|---|
| 12,19 | Singulet large | 1 | - | NH⁺ |
| 8,03 | Doublet | 2 | ³J_{H-H}≈ 9,0 | H(2) et H(2') |
| 6,91 | Doublet | 2 | ³J_{H-H}≈ 9, 0 | H(3) et H(3') |
| 4,18 | Triplet | 2 | ³J_{H-H}≈ 5,5 | OCH₂(5) |
| 3 , 21 | Multiplet | 2 | - | CH₂(7) |
| 3,02 | Multiplet | 4 | - | CH₂(8) et CH₂(8') |
| 2,42 | Multiplet | 2 | - | CH₂(6) |
| 1,79 | Multiplet | 4 | - | CH₂(9) et CH₂(9') |
| 1,38 | Sextuplet | 4 | - | CH₂(10) et CH₂(10') |
| 0,95 | Triplet | 6 | - | CH₃(11) et CH₃(11') |

### EXEMPLE 4

### Chlorhydrate de 2-butyl-3-(4-[3-(dibutylamino)propoxy]benzoyl)-5-nitro-benzofurane

Dans un ballon, on introduit le chlorhydrate du chlorure de 4-[3-(dibutylamino)propoxy]benzoyle obtenu à l'Exemple 3 précédent puis on ajoute 35,1 g (0,160 mole) de 2-butyl-5-nitro-benzofurane. On refroidit le milieu entre 0 et 3°C puis on introduit 66,9 g (0,4 mole) de chlorure ferrique en maintenant un refroidissement par bain de glace/méthanol. On maintient la température à 20 à 22°C pendant 1 heure 30 minutes puis on hydrolyse le milieu par ajout de 400 ml d'eau déminéralisée en maintenant la température du milieu entre 20 et 25°C. On décante durant 30 minutes et on élimine la phase aqueuse supérieure. Au moyen de 300 ml d'eau désionisée on effectue ensuite 5 lavages de la phase organique.

De cette manière, on obtient le composé souhaité sous forme d'une huile.
Rendement : environ 98 %.

### EXEMPLE 5

### 2-Butyl-3-(4-[3-(dibutylamino)propoxy]benzoyl)-5-nitro-benzofurane

Dans un ballon de 1 I, on introduit 126,6 g (0,368 mole) de chlorhydrate de l'acide 4-[3-dibutylamino)propoxy]benzoïque, 600 ml de chlorobenzène et 3 gouttes de N,N-diméthylformamide. Tout en maintenant le mélange sous atmosphère inerte, on introduit, en environ 45 minutes, 87,6 g (0,736 mole) de chlorure de thionyle. On maintient durant 1 heure à 85 ± 1°C pour former le chlorhydrate du chlorure de 4-[3-(dibutylamino)propoxy]benzoyle puis on distille sous vide progressif, 225 g d'un mélange de chlorobenzène et de chlorure de thionyle. On refroidit le milieu réactionnel à température ambiante (20 à 22°C) puis on ajoute 70,2 g (0,320 mole) de 2-butyl-5-nitro-benzofurane. On refroidit le milieu entre 0 et 3°C puis on introduit 133,8 g (0,8 mole) de chlorure ferrique en maintenant un refroidissement par bain de glace/méthanol.

La température s'élève à environ 20°C et on l'y maintient (20 à 22°C) pendant 1 heure 30 minutes. On hydrolyse le milieu réactionnel par ajout de 800 ml d'eau déminéralisée en maintenant la température du milieu entre 20 et 25°C. On décante durant 30 minutes et on élimine la phase aqueuse supérieure.

Au moyen de 600 ml d'eau désionisée on effectue ensuite 5 lavages de la phase organique contenant le chlorhydrate de 2-butyl-3-(4-[3-(dibutylamino)propoxy]benzoyl)-5-nitro-benzofurane ainsi formé. On effectue un sixième lavage avec 600 ml d'eau désionisée contenant 50 g (0,595 mole) d'hydrogénocarbonate sodique pour libérer le composé désiré. On élimine le chlorobenzène à l'évaporateur rotatif (température du bain : 35°C, pression : 10 mmHg (1333,22 Pa)).

De cette manière, on obtient 192 g de composé souhaité.
Rendement : environ 95 %.
Pureté (CLHP)
   Composé désiré : 98,3 %
   Acide 4-[3-(dibutylamino)propoxy]benzoïque : 0,9 %
   Titre : 80 à 82 %

### EXEMPLE 6

### Chlorhydrate de dronédarone

### A. 5-Amino-2-butyl-3-(4-[3-(dibutylamino)propoxy]benzoyl) -benzofurane

Dans un appareil à hydrogéner on agite, sous pression de 3,4 atmosphères (3,44 x 10⁵ Pa) d'hydrogène, un milieu formé de 20,4 g (0,04 mole) de 2-butyl-3-(4-[3-(dibutylamino)propoxy]benzoyl)-5-nitro-benzofurane préparé comme décrit à l'Exemple 5, 200 ml d'éthanol et 0,6g d'oxyde de platine. Lorsque la pression atteint 2,7 atm (2,73 x 10⁵ Pa), la réaction est terminée, ce qui nécessite environ 20 minutes.

De cette manière on obtient le 5-amino-2-butyl-3-(4-[3-(dibutylamino)propoxy]benzoyl-benzofurane.
Rendement : 98,4 %.
Pureté (CLHP) : 95,28 %.

### B. Dronédarone

A une solution de 68,3 g (0,15 mole) de composé obtenu au paragraphe **A.** précédent et de 23,6 g (0,23 mole) de triéthylamine dans 750 ml de dichloréthane, on ajoute, goutte à goutte une solution de 17,6 g (0,154 mole) de chlorure de méthanesulfonyle dans 375 ml de dichloréthane. On agite durant 20 heures et on verse dans 500 ml d'eau. On décante, on lave à l'eau et on évapore à sec. On purifie par chromatographie d'elution sur colonne de silice (éluant : acitate d'éthyle), le produit brut ainsi obtenu (79,5 g ; rendement brut : 100%).

De cette manière, on recueille 48 g de dronédarone purifiée. Rendement : 61,6 %.

Un traitement par l'hexane du produit ainsi obtenu a fourni une fraction de 44 g à l'état cristallin (pureté par CLHP : 96,1 %) et une fraction de 4 g à l'état cristallin (pureté par CLHP : 99%).
Rendement : 65,3 %.

### C. Chlorhydrate de dronédarone

On dissout 2 g de dronédarone dans 40 ml d'acétate d'éthyle anhydre. Tout en agitant, on ajoute de l'éther chlorhydrique jusqu'à pH=3. Après quelques minutes, le chlorhydrate commence à précipiter. On le filtre après 0,75 heure, ce qui fournit 2,03 g d'un produit blanc.

De cette manière, on recueille le chlorhydrate de drondédarone. P.F. : 143°C (acétone)

## Revendications

1. Procédé de préparation du chlorhydrate de 2-butyl-3-(4-[3-(dibutylamino)propoxy]benzoyl)-5-nitro-benzofurane, **caractérisé en ce que**, dans une phase organique constituée d'un ou de plusieurs solvants choisis parmi des hydrocarbures halogénés ou non, on fait réagir le 2-butyl-5-nitro-benzofurane avec le chlorhydrate du chlorure de 4-[3-(dibutylamino)propoxy]benzoyle et ce, en présence d'un maximum de 3 équivalents molaires d'un acide de Lewis comme catalyseur puis on hydrolyse pour former le composé désiré que l'on récupère dans la phase organique.

2. Procédé de préparation du 2-butyl-3-(4-[3-(dibutylamino)propoxy]benzoyl)-5-nitro-benzofurane, **caractérisé en ce que** l'on prépare du chlorhydrate de 2-butyl-3-(4-[3-(dibutylamino)propoxy]benzoyl)-5-nitro-benzofurane, par le procédé selon la revendication 1, et **en ce que** l'on traite le composé ainsi obtenu au moyen d'un agent basique, ce qui fournit le composé désiré.

3. Procédé de préparation du 2-butyl-3-(4-[3-(dibutylamino)propoxy]benzoyl)-5-nitro-benzofurane, **caractérisé en ce que** dans une phase organique, constituée d'un ou de plusieurs solvants choisis parmi des hydrocarbures halogénés ou non, on fait réagir le 2-butyl-5-nitro-benzofurane avec le chlorhydrate du chlorure de 4-[3-(dibutylamino)propoxy]benzoyle et ce, en présence d'un maximum de 3 équivalents molaires d'un acide de Lewis comme catalyseur puis on hydrolyse pour obtenir transitoirement et sans isolement, le chlorhydrate de 2-butyl-3-(4-[3-(dibutylamino)propoxy]benzoyl-5-nitro-benzofurane que l'on récupère dans la phase organique et que l'on traite au moyen d'un agent basique, ce qui fournit le composé désiré.

4. Procédé de préparation de la dronédarone et de ses sels pharmaceutiquement acceptables, **caractérisé en ce que** :
a)-(i) dans une phase organique constituée d'un ou de plusieurs solvants choisis parmi des hydrocarbures halogénés ou non, on fait réagir le 2-butyl-5-nitro-benzofurane avec le chlorhydrate du chlorure
de 4-[3-(dibutylamino)propoxy]benzoyle et ce, en présence d'un maximum de 3 équivalents molaires d'un acide de Lewis comme catalyseur puis on hydrolyse pour former le chlorhydrate de 2-butyl-3-(4-[3-(dibutylamino)propoxy]benzoyl)-5-nitro-benzofurane,
a)-ii) on traite le chlorhydrate de 2-butyl-3-(4-[3-(dibutylamino)propoxy]benzoyl)-5-nitro-benzofurane au moyen d'un agent basique, ce qui fournit le 2-butyl-3-(4-[3-(dibutylamino)propoxy]benzoyl)-5-nitro-benzofurane,
b) l'on hydrogène le composé ainsi obtenu en présence d'un catalyseur approprié, ce qui fournit le 5-amino-2-butyl-3-(4-[3-(dibutylamino)propoxy]benzoyl-benzofurane,
c) l'on fait réagir le dérivé de 5-aminobenzofurane ainsi obtenu avec le chlorure de méthanesulfonyle ou l'anhydride méthanesulfonique, la réaction ayant lieu en présence d'un accepteur d'acide, ce qui fournit la dronédarone que l'on peut faire réagir, si on le désire, avec un acide organique ou inorganique pour former un sel pharmaceutiquement acceptable de ce composé.

5. Procédé de préparation de la dronédarone et de ses sels pharmaceutiquement acceptables, **caractérisé en ce que** :
a) dans une phase organique, constituée d'un ou de plusieurs solvants choisis parmi des hydrocarbures halogénés ou non, on fait réagir le 2-butyl-5-nitro-benzofurane avec le chlorhydrate du chlorure de 4-[3-(dibutylamino)propoxy]benzoyle et ce, en présence d'un maximum de 3 équivalents molaires d'un acide de Lewis comme catalyseur puis on hydrolyse pour obtenir transitoirement et sans isolement, le chlorhydrate de 2-butyl-3-(4-[3-dibutylamino)propoxy]benzoyl-5-nitro-benzofurane que l'on récupère dans la phase organique et que l'on traite au moyen d'un agent basique pour former le 2-butyl-3-(4-[3-(dibutylamino)propoxy]benzoyl)-5-nitro-benzofurane,
b) l'on hydrogène le composé ainsi obtenu en présence d'un catalyseur approprié, ce qui fournit le 5-amino-2-butyl-3-(4-[3-(dibutylamino)propoxy]benzoyl-benzofurane,
c) l'on fait réagir le dérivé de 5-aminobenzofurane ainsi obtenu avec le chlorure de méthanesulfonyle ou l'anhydride méthanesulfonique, la réaction ayant lieu en présence d'un accepteur d'acide, ce qui fournit la dronédarone que l'on peut faire réagir, si on le désire, avec un acide organique ou inorganique pour former un sel pharmaceutiquement acceptable de ce composé.

6. Procédé selon la Revendication 1, 3, 4 ou 5 **caractérisé en ce que** la phase organique est constituée d'un ou de plusieurs solvants choisis parmi des hydrocarbures halogénés de type aliphatique, alicyclique ou aromatique.

7. Procédé selon la revendication 1, 3, 4 ou 5, **caractérisé en ce que** l'acide de Lewis est choisi dans le groupe constitué par le chlorure d'aluminium, le chlorure de zinc, le trifluorure de bore, le chlorure stannique, le tétrachlorure de titane, le chlorure ferrique et les mélanges de ceux-ci.

8. Procédé selon la Revendication 1, 3, 4, 5 ou 7, **caractérisé en ce que** l'acide de Lewis est le chlorure ferrique.

9. Procédé selon une des Revendications 1, 3, 4, 5, 7 ou 8, **caractérisé en ce que** l'acide de Lewis est utilisé à raison de 2 à 3 équivalents molaires.

10. Procédé selon une des Revendications 1, 3, 4 ou 5, **caractérisé en ce que** le chlorhydrate du chlorure de 4-[3-(dibutylamino)propoxy] benzoyle est utilisé selon des quantités de l'ordre de 1 à 1,3 équivalent molaire.

11. Procédé selon une des Revendications 1, 3, 4 ou 5, **caractérisé en ce que** l'agent basique est l'hydrogénocarbonate de sodium ou l'hydroxyde de sodium.

## Claims

1. Process for the preparation of 2-butyl-3-(4-[3-(dibutylamino)propoxy]benzoyl)-5-nitrobenzofuran hydrochloride, **characterized in that**, in an organic phase composed of one or more solvents chosen from halogenated or nonhalogenated hydrocarbons, 2-butyl-5-nitrobenzofuran is reacted with 4-[3-(dibutylamino)propoxy]benzoyl chloride hydrochloride, this taking place in the presence of a maximum of 3 molar equivalents of a Lewis acid as catalyst, and then hydrolysis is carried out to form the desired compound, which is recovered in the organic phase.

2. Process for the preparation of 2-butyl-3-(4-[3-(dibutylamino)propoxy]benzoyl)-5-nitrobenzofuran, **characterized in that** 2-butyl-3-(4-[3-(dibutyl-amino)propoxy]benzoyl)-5-nitrobenzofuran hydrochloride is prepared by the process according to Claim 1 and **in that** the compound thus obtained is treated using a basic agent, which gives the desired compound.

3. Process for the preparation of 2-butyl-3-(4-[3-(dibutylamino)propoxy]benzoyl)-5-nitrobenzofuran, **characterized in that**, in an organic phase composed of one or more solvents chosen from halogenated or nonhalogenated hydrocarbons, 2-butyl-5-nitrobenzofuran is reacted with 4-[3-(dibutylamino)propoxy]benzoyl chloride hydrochloride, this taking place in the presence of a maximum of 3 molar equivalents of a Lewis acid as catalyst, and then hydrolysis is carried out to produce 2-butyl-3-(4-[3-(dibutylamino)propoxy]-benzoyl)-5-nitrobenzofuran hydrochloride as an intermediate and without isolation, which product is recovered in the organic phase and which is treated using a basic agent, which gives the desired compound.

4. Process for the preparation of dronedarone and of its pharmaceutically acceptable salts, **characterized in that**:
a)-(i) in an organic phase composed of one or more solvents chosen from halogenated or nonhalogenated hydrocarbons, 2-butyl-5-nitrobenzofuran is reacted with 4-[3-(dibutylamino)propoxy]benzoyl chloride hydrochloride, this taking place in the presence of a maximum of 3 molar equivalents of a Lewis acid as catalyst, and then hydrolysis is carried out to form 2-butyl-3-(4-[3-(dibutylamino)-propoxy)benzoyl)-5-nitrobenzofuran hydrochloride,
a)-ii) 2-butyl-3-(4-[3-(dibutylamino)propoxy]-benzoyl)-5-nitrobenzofuran hydrochloride is treated using a basic agent, which gives 2-butyl-3-(4-[3-dibutylamino)propoxy]benzoyl)-5-nitrobenzofuran,
b) the compound thus obtained is hydrogenated in the presence of an appropriate catalyst, which gives 5-amino-3-butyl-3-(4-[3-(dibutylamino)-propoxy]benzoyl)benzofuran,
c) the 5-aminobenzofuran derivative thus obtained is reacted with methanesulphonyl chloride or methanesulphonic anhydride, the reaction taking place in the presence of an acid acceptor, which gives dronedarone, which can be reacted, if desired, with an organic or inorganic acid to form a pharmaceutically acceptable salt of this compound.

5. Process for the preparation of dronedarone and its pharmaceutically acceptable salts, **characterized in that**:
a) in an organic phase composed of one or more solvents chosen from halogenated or nonhalogenated hydrocarbons, 2-butyl-5-nitrobenzofuran is reacted with 4-[3-(dibutylamino)propoxy]benzoyl chloride hydrochloride, this taking place in the presence of a maximum of 3 molar equivalents of a Lewis acid as catalyst, and then hydrolysis is carried out to produce 2-butyl-3-(4-[3-(dibutylamino)-propoxy]benzoyl)-5-nitrobenzofuran hydrochloride as an intermediate and without isolation, which product is recovered in the organic phase and which is treated using a basic agent to form 2-butyl-3-(4-[3-(dibutylamino)propoxy]benzoyl)-5-nitrobenzofuran,
b) the compound thus obtained is hydrogenated in the presence of an appropriate catalyst, which gives 5-amino-2-butyl-3-(4-[3-(dibutylamino)-propoxy]benzoyl)benzofuran,
c) the 5-aminobenzofuran derivative thus obtained is reacted with methanesulphonyl chloride or methanesulphonic anhydride, the reaction taking place in the presence of an acid acceptor, which gives dronedarone, which can be reacted, if desired, with an organic or inorganic acid to form a pharmaceutically acceptable salt of this compound.

6. Process according to Claim 1, 3, 4 or 5, **characterized in that** the organic phase is composed of one or more solvents chosen from halogenated hydrocarbons of aliphatic, alicyclic or aromatic type.

7. Process according to Claim 1, 3, 4 or 5, **characterized in that** the Lewis acid is chosen from the group consisting of aluminium chloride, zinc chloride, boron trifluoride, stannic chloride, titanium tetrachloride, ferric chloride and the mixtures of these.

8. Process according to Claim 1, 3, 4, 5 or 7, **characterized in that** the Lewis acid is ferric chloride.

9. Process according to one of Claims 1, 3, 4, 5, 7 or 8, **characterized in that** the Lewis acid is used in a proportion of 2 to 3 molar equivalents.

10. Process according to one of Claims 1, 3, 4 or 5, **characterized in that** 4-[3-(dibutylamino)-propoxy]benzoyl chloride hydrochloride is used in amounts of the order of 1 to 1.3 molar equivalents.

11. Process according to one of Claims 1, 3, 4 or 5, **characterized in that** the basic agent is sodium hydrogen- carbonate or sodium hydroxide.

## Patentansprüche

1. Verfahren zur Herstellung von 2-Butyl-3-(4-[3-(dibutylamino)propoxy]benzoyl)-5-nitrobenzofuran-hydrochlorid, **dadurch gekennzeichnet, daß** man 2-Butyl-5-nitrobenzofuran mit 4-[3-(Dibutylamino)-propoxy]benzoylchlorid-hydrochlorid in Gegenwart von maximal drei Moläquivalenten einer Lewis-Säure als Katalysator in einer organischen Phase, die aus einem oder mehreren Lösungsmitteln aus der Reihe der gegebenenfalls halogenierten Kohlenwasserstoffe besteht, umsetzt und das Umsetzungsprodukt anschließend zu der gewünschten Verbindung hydrolysiert, die man aus der organischen Phase gewinnt.

2. Verfahren zur Herstellung von 2-Butyl-3-(4-[3-(dibutylamino)propoxy]benzoyl)-5-nitrobenzofuran, **dadurch gekennzeichnet, daß** man mit dem Verfahren nach Anspruch 1 das 2-Butyl-3-(4-[3-(dibutylamino)propoxy]benzoyl)-5-nitrobenzofuran-hydrochlorid herstellt und die so erhaltene Verbindung mit einem basischen Mittel behandelt, wodurch man zu der gewünschten Verbindung gelangt.

3. Verfahren zur Herstellung von 2-Butyl-3-(4-[3-(dibutylamino)propoxy]benzoyl]-5-nitrobenzofuran, **dadurch gekennzeichnet, daß** man 2-Butyl-5-nitrobenzofuran mit 4-[3-(Dibutylamino)propoxy]benzoylchlorid-hydrochlorid in Gegenwart von maximal drei Moläquivalenten einer Lewis-Säure als Katalysator in einer organischen Phase, die aus einem oder mehreren Lösungsmitteln aus der Reihe der gegebenenfalls halogenierten Kohlenwasserstoffe besteht, umsetzt und das Umsetzungsprodukt anschließend hydrolysiert, um übergangsmäßig und ohne Isolation zum 2-Butyl-3-(4-[3-(dibutylamino)propoxy]benzoyl)-5-nitrobenzofuran-hydrochlorid zu gelangen, das man aus der organischen Phase gewinnt und mit einem basischen Mittel behandelt, wodurch man zu der gewünschten Verbindung gelangt.

4. Verfahren zur Herstellung von Dronedaron und seinen pharmazeutisch unbedenklichen Salzen, **dadurch gekennzeichnet, daß** man:
a)-(i) 2-Butyl-5-nitrobenzofuran mit 4-[3-(Dibutylamino)propoxy]benzoylchlorid-hydrochlorid in Gegenwart von maximal drei Moläquivalenten einer Lewis-Säure als Katalysator in einer organischen Phase, die aus einem oder mehreren Lösungsmitteln aus der Reihe der gegebenenfalls halogenierten Kohlenwasserstoffe besteht, umsetzt und das Umsetzungsprodukt anschließend zu 2-Butyl-3-(4-[3-(dibutylamino)propoxy]benzoyl)-5-nitrobenzofuran-hydrochlorid hydrolysiert,
a)-(ii) 2-Butyl-3-(4-[3-(dibutylamino)propoxy]benzoyl)-5-nitrobenzofuran-hydrochlorid mit einem basischen Mittel behandelt, wodurch man zu 2-Butyl-3-(4-[3-dibutylamino)propoxy]benzoyl)-5-nitrobenzofuran gelangt,
b) die oben erhaltene Verbindung in Gegenwart eines entsprechenden Katalysators hydriert, wodurch man zu 5-Amino-2-butyl-3-(4-[3-(dibutylamino)propoxy]benzoyl)benzofuran gelangt,
c) das so erhaltene 5-Aminobenzofuranderivat mit Methansulfonylchlorid oder Methansulfonsäureanhydrid in Gegenwart eiens Säureakzeptors umsetzt, wodurch man zum Dronedaron gelangt, das man gewünschtenfalls mit einer organischen oder anorganischen Säure zu einem pharmazeutisch unbedenklichen Salz dieser Verbindung umsetzt.

5. Verfahren zur Herstellung von Dronedaron und seinen pharmazeutisch unbedenklichen Salzen, **dadurch gekennzeichnet, daß** man
a) 2-Butyl-5-nitrobenzofuran mit 4-[3-(Dibutylamino)propoxy]benzoylchlorid-hydrochlorid in Gegenwart von maximal drei Moläquivalenten einer Lewis-Säure als Katalysator in einer organischen Phase, die aus einem oder mehreren Lösungsmitteln aus der Reihe der gegebenenfalls halogenierten Kohlenwasserstoffe besteht, umsetzt und das Umsetzungsprodukt anschließend hydrolysiert, um übergangsmäßig und ohne Isolation zum 2-Butyl-3-(4-[3-(dibutylamino)propoxy]benzoyl)-5-nitrobenzofuran-hydrochlorid zu gelangen, das man aus der organischen Phase gewinnt und mit einem basischen Mittel behandelt, wodurch man 2-Butyl-3-(4-[3-(dibutylamino)propoxy]benzoyl)-5-nitrobenzofuran erhält,
b) die oben erhaltene Verbindung in Gegenwart eines entsprechenden Katalysators hydriert, wodurch man zu 5-Amino-3-butyl-3-(4-[3-(dibutylamino)propoxy]benzoyl)benzofuran gelangt,
c) das so erhaltene 5-Aminobenzofuranderivat mit Methansulfonylchlorid oder Methansulfonsäureanhydrid in Gegenwart eines Säureakzeptors umsetzt, wodurch man zum Dronedaron gelangt, das man gewünschtenfalls mit einer organischen oder anorganischen Säure zu einem pharmazeutisch unbedenklichen Salz dieser Verbindung umsetzt.

6. Verfahren nach Anspruch 1, 3, 4 oder 5, **dadurch gekennzeichnet, daß** die organische Phase aus einem oder mehreren Lösungsmitteln erster Reihe der halogenierten Kohlenwasserstoffe des aliphatischen, alicyclischen oder aromatischen Typs besteht.

7. Verfahren nach Anspruch 1, 3, 4 oder 5, **dadurch gekennzeichnet, daß** die Lewis-Säure aus der Gruppe Aluminiumchlorid, Zinkchlorid, Bortrifluorid, Zinn(IV)-chlorid, Titantetrachlorid, Eisen(III)-chlorid und deren Mischungen stammt.

8. Verfahren nach Anspruch 1, 3, 4, 5 oder 7, **dadurch gekennzeichnet, daß** es sich bei der Lewis-Säure um Eisen(III)-chlorid handelt.

9. Verfahren nach einem der Ansprüche 1, 3, 4, 5, 7 oder 8, **dadurch gekennzeichnet, daß** man die Lewis-Säure in einer Menge von 2-3 Moläquivalenten verwendet.

10. Verfahren nach einem der Ansprüche 1, 3, 4 oder 5, **dadurch gekennzeichnet, daß** man das 4-[3-(Dibutylamino)propoxy]benzoylchlorid-hydrochlorid in Mengen in einer Größenordnung von 1 bis 1,3 Moläquivalent verwendet.

11. Verfahren nach einem der Ansprüche 1, 3, 4 oder 5, **dadurch gekennzeichnet, daß** es sich bei dem basischen Mittel um Natriumhydrogencarbonat oder Natriumhydroxid handelt.
